# EUROPEAN PATENT APPLICATION

(11) **EP 4 653 835 A1**
(43) Date of publication of application: **26.11.2025**
(21) Application number: 25176637.4
(22) Date of filing: 15.05.2025
(51) Int. Cl.: G01L 19/02, A61B 5/11, G01L 27/00, G08B 21/04

(54) **DEVICE AND METHOD FOR ADJUSTING PRESSURE SENSOR DATA**

(30) Priority: 24.05.2024 US 202418673813
(71) Applicant: STMicroelectronics International N.V., 1228 Plan-les-Ouates, Geneva (CH)
(72) Inventor: WON, Hyeok, 16108 UI-WANG (KR); KANG, Tae-gil, 05646 SEOUL (KR); KANG, Sinwon, 06611 SEOUL (KR)
(74) Representative: Cabinet Beaumont

(57) **Abstract**

In accordance with various embodiments of the present disclosure, a device is provided that comprises a pressure sensor, an accelerometer, and a controller. The controller is configured to determine a tilt angle of the device, receive an unadjusted pressure value from the pressure sensor, determine a pressure adjustment value based on (a) the tilt angle and (b) a slope value of pressure test data taken at a first test angle and at a second test angle different than the first test angle, and determine an adjusted pressure value by adding the determined pressure adjustment value to the received unadjusted pressure value.

## Description

### FIELD OF THE INVENTION

Example embodiments of the present disclosure relate generally to pressure sensors and, more particularly, to waterproof pressure sensors.

### BACKGROUND

Pressure sensors are commonly used in mobile and wearable devices, such as mobile phones and smart watches. Such pressure sensors are used to determine air pressure, which may be used to determine the altitude of the device (and therefore of a user of the device). Such an air pressure determination may also be used to detect when a user has fallen by detecting very small changes in pressure (e.g., about 0.1 hectopascals (hPa)) which correspond to very small changes in altitude (e.g., about 80 centimeters).

Such pressure sensors are also used for measuring water pressure. The measured water pressure can be used for determining a depth under water, which is useful for divers or swimmers. For this reason, mobile and wearable devices are more commonly being waterproofed, which in turn requires the use of waterproof pressure sensors. Such waterproof pressure sensors commonly use a gel inside the sensor to protect the electronics from exposure to water.

However, when the device is turned over or tilted, movement also occurs in the gel which can influence the membrane of the pressure sensor and cause a drift in the pressure sensing ability. The drift of pressure data can cause a malfunction of some algorithms that use the pressure data, such as a fall detection algorithm.

Applicant has identified many technical challenges and difficulties associated with the use of waterproof sensors in mobile and wearable devices. Through applied effort, ingenuity, and innovation, Applicant has solved problems related to the use of such waterproof sensors by developing solutions embodied in the present disclosure, which are described in detail below.

### BRIEF SUMMARY

Various embodiments described herein related to devices and methods for adjusting sensor data from waterproof sensors.

In accordance with various embodiments of the present disclosure, a device is provided that comprises a pressure sensor, an accelerometer, and a controller. The controller is configured to determine a tilt angle of the device, receive an unadjusted pressure value from the pressure sensor, determine a pressure adjustment value based on (a) the tilt angle and (b) a slope value of pressure test data taken at a first test angle and at a second test angle different than the first test angle, and determine an adjusted pressure value by adding the determined pressure adjustment value to the received unadjusted pressure value.

In some embodiments, the controller determines the tilt angle of the device using data received from the accelerometer.

In some embodiments, the slope value is based on an average of a plurality of pressure test readings taken at the first test angle and on an average of a plurality of pressure test readings taken at the second test angle.

In some embodiments, the slope value is calculated as (the average of the plurality of pressure test readings taken at the second test angle minus the average of the plurality of pressure test readings taken at the first test angle) divided by a difference in degrees between the second test angle and the first test angle.

In some embodiments, the controller is further configured to repeatedly determine the tilt angle of the device, receive the unadjusted pressure value from the pressure sensor, determine the pressure adjustment value based on (a) the tilt angle and (b) the slope value of pressure test data taken at the first test angle and at the second test angle, and determine the adjusted pressure value by adding the determined pressure adjustment value to the received unadjusted pressure value.

In some embodiments, the controller is further configured to determine if a user of the device has fallen based on changes in repeated determinations of the adjusted pressure value.

In some embodiments, the controller determines that the user of the device has fallen if the changes in repeated determinations of the adjusted pressure value exceed a predetermined threshold.

In some embodiments, the device comprises a wearable device.

In some embodiments, the first test angle and the second test angle are 180 degrees apart.

In some embodiments, the first test angle is zero degrees and the second test angle is 180 degrees.

In accordance with various embodiments of the present disclosure, a method of adjusting a pressure reading from a pressure sensor in a device is provided. In some embodiments, the method comprises determining a tilt angle of the device containing the pressure sensor; receiving an unadjusted pressure value from the pressure sensor; determining a pressure adjustment value based on (a) the tilt angle and (b) a slope value of pressure test data taken at a first test angle and at a second test angle different than the first test angle; and determining an adjusted pressure value by adding the determined pressure adjustment value to the received unadjusted pressure value.

The above summary is provided merely for purposes of summarizing some example embodiments to provide a basic understanding of some aspects of the disclosure. Accordingly, it will be appreciated that the above-described embodiments are merely examples and should not be construed to narrow the scope or spirit of the disclosure in any way. It will also be appreciated that the scope of the disclosure encompasses many potential embodiments in addition to those here summarized, some of which will be further described below.

### BRIEF DESCRIPTION OF THE DRAWINGS

The description of the illustrative embodiments may be read in conjunction with the accompanying figures. It will be appreciated that, for simplicity and clarity of illustration, elements illustrated in the figures have not necessarily been drawn to scale, unless described otherwise. For example, the dimensions of some of the elements may be exaggerated relative to other elements, unless described otherwise. Embodiments incorporating teachings of the present disclosure are shown and described with respect to the figures presented herein, in which:
FIG. 1 provides a block diagram of an example device for adjusting sensor data from waterproof sensors, in accordance with some embodiments of the present disclosure; and
FIG. 2 provides an example flow diagram illustrating an example method for adjusting sensor data from waterproof sensors, in accordance with some embodiments of the present disclosure.

### DETAILED DESCRIPTION OF THE INVENTION

Some embodiments of the present disclosure will now be described more fully hereinafter with reference to the accompanying drawings, in which some, but not all embodiments of the disclosure are shown. Indeed, these disclosures may be embodied in many different forms and should not be construed as limited to the embodiments set forth herein; rather, these embodiments are provided so that this disclosure will satisfy applicable legal requirements. Like numbers refer to like elements throughout.

As used herein, terms such as "front," "rear," "top," etc. are used for explanatory purposes in the examples provided below to describe the relative position of certain components or portions of components. Furthermore, as would be evident to one of ordinary skill in the art in light of the present disclosure, the terms "substantially" and "approximately" indicate that the referenced element or associated description is accurate to within applicable engineering tolerances.

As used herein, the term "comprising" means including but not limited to and should be interpreted in the manner it is typically used in the patent context. Use of broader terms such as comprises, includes, and having should be understood to provide support for narrower terms such as consisting of, consisting essentially of, and comprised substantially of.

The phrases "in one embodiment," "according to one embodiment," and the like generally mean that the particular feature, structure, or characteristic following the phrase may be included in at least one embodiment of the present disclosure, and may be included in more than one embodiment of the present disclosure (importantly, such phrases do not necessarily refer to the same embodiment).

The word "example" or "exemplary" is used herein to mean "serving as an example, instance, or illustration." Any implementation described herein as "exemplary" is not necessarily to be construed as preferred or advantageous over other implementations.

If the specification states a component or feature "may," "can," "could," "should," "would," "preferably," "possibly," "typically," "optionally," "for example," "often," or "might" (or other such language) be included or have a characteristic, that a specific component or feature is not required to be included or to have the characteristic. Such a component or feature may be optionally included in some embodiments, or it may be excluded.

Various embodiments of the present disclosure overcome the above technical challenges and difficulties and provide various technical improvements and advantages based on, for example, but not limited to, providing example devices in which the pressure reading from a waterproof pressure sensor is adjusted to compensate for the drift in the pressure sensing ability caused by tilting the device. In some embodiments, such devices comprise mobile devices (e.g., mobile phones), wearable devices (e.g., smart watches), or any other suitable devices.

In various embodiments, the pressure reading from a waterproof pressure sensor is adjusted using a pressure adjustment value. In various embodiments, such a pressure adjustment value is calculated using a slope determined during testing using average pressure readings at a zero degree tilt angle and average pressure readings at a 180 degree tilt angle.

In various embodiments, a waterproof sensor, such as a sensor with a gel protecting the electronics of the sensor, is tested to obtain pressure readings at two different tilt angles. In various embodiments, the two different tilt angles are zero degrees (i.e., upright) and 180 degrees (i.e., upside down). In some embodiments, tilt angles other than zero degrees and 180 degrees can be used. In various embodiments, the pressure sensor testing is performed before the pressure sensor is assembled into a device and/or after the pressure sensor is assembled into a device.

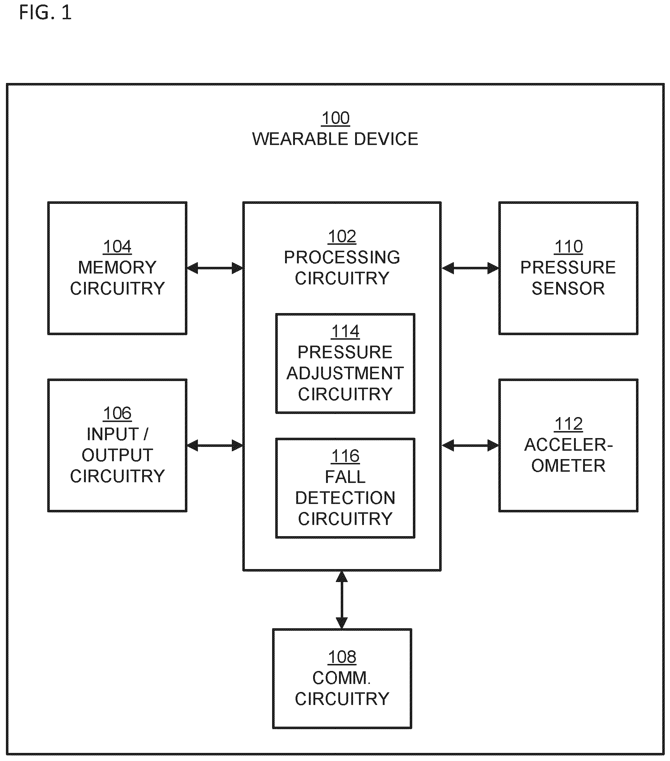

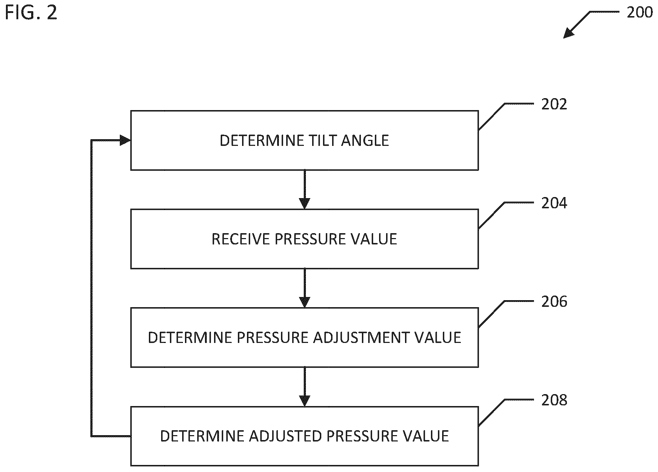

FIG. 1 illustrates an exemplary block diagram of an example device that may be specially configured in accordance with an example embodiment of the present disclosure. Specifically, FIG. 1 depicts an example wearable device 100 specially configured in accordance with at least some example embodiments of the present disclosure. The device 100 of FIG. 1 comprises processing circuitry 102, memory circuitry 104, input/output circuitry 106, communications circuitry 108, a pressure sensor 110 (such as a waterproof pressure sensor), and an accelerometer 112. In the illustrated embodiment, the processing circuitry includes pressure adjustment circuitry 114 and fall detection circuitry 116. In some embodiments, the device 100 is configured to execute and perform the operations described herein. For example, the device 100 may be configured to implement a method for adjusting sensor data from waterproof sensors based on a tilt angle of the device as described below in relation to FIG. 2.

Although components are described with respect to functional limitations, it should be understood that the particular implementations necessarily include the use of particular computing hardware. It should also be understood that in some embodiments certain of the components described herein include similar or common hardware. For example, in some embodiments two sets of circuitry both leverage use of the same processor(s), memory(ies), circuitry(ies), and/or the like to perform their associated functions such that duplicate hardware is not required for each set of circuitry.

Processing circuitry 102 may be embodied in a number of different ways. In various embodiments, the use of the terms "processor," "processing circuitry," "controller," or "control circuitry" should be understood to include a single core processor, a multi-core processor, multiple processors internal to the device 100, and/or one or more remote or "cloud" processor(s) external to the device 100. In some example embodiments, processing circuitry 102 may include one or more processing devices configured to perform independently. Alternatively, or additionally, processing circuitry 102 may include one or more processor(s) configured in tandem via a bus to enable independent execution of operations, instructions, pipelining, and/or multithreading.

In an example embodiment, the processing circuitry 102 may be configured to execute instructions stored in the memory circuitry 104 or otherwise accessible to the processor. Alternatively, or additionally, the processing circuitry 102 may be configured to execute hard-coded functionality. As such, whether configured by hardware or software methods, or by a combination thereof, processing circuitry 102 may represent an entity (e.g., physically embodied in circuitry) capable of performing operations according to embodiments of the present disclosure while configured accordingly. Alternatively, or additionally, processing circuitry 102 may be embodied as an executor of software instructions, and the instructions may specifically configure the processing circuitry 102 to perform the various algorithms embodied in one or more operations described herein when such instructions are executed. In some embodiments, the processing circuitry 102 includes hardware, software, firmware, and/or a combination thereof that performs one or more operations described herein.

In some embodiments, the processing circuitry 102 (and/or co-processor or any other processing circuitry assisting or otherwise associated with the processor) is/are in communication with the memory circuitry 104 via a bus for passing information among components of the device 100.

Memory or memory circuitry 104 may be non-transitory and may include, for example, one or more volatile and/or non-volatile memories. In some embodiments, the memory circuitry 104 includes or embodies an electronic storage device (e.g., a computer readable storage medium). In some embodiments, the memory circuitry 104 is configured to store information, data, content, applications, instructions, or the like, for enabling a device 100 to carry out various operations and/or functions in accordance with example embodiments of the present disclosure.

Input/output circuitry 106 may be included in the device 100. In some embodiments, input/output circuitry 106 may provide output to the user and/or receive input from a user. The input/output circuitry 106 may be in communication with the processing circuitry 102 to provide such functionality. The input/output circuitry 106 may comprise one or more user interface(s). In some embodiments, a user interface may include a display that comprises the interface(s) rendered as a web user interface, an application user interface, a user device, a backend system, or the like. In some embodiments, the input/output circuitry 106 also includes a keyboard, a mouse, a joystick, a touch screen, touch areas, soft keys a microphone, a speaker, or other input/output mechanisms. The processing circuitry 102 and/or input/output circuitry 106 may be configured to control one or more operations and/or functions of one or more user interface elements through computer program instructions (e.g., software and/or firmware) stored on a memory accessible to the processor (e.g., memory circuitry 104, and/or the like). In some embodiments, the input/output circuitry 106 includes or utilizes a user-facing application to provide input/output functionality to a computing device and/or other display associated with a user.

Communications circuitry 108 may be included in the device 100. The communications circuitry 108 may include any means such as a device or circuitry embodied in either hardware or a combination of hardware and software that is configured to receive and/or transmit data from/to a network and/or any other device, circuitry, or module in communication with the device 100. In some embodiments the communications circuitry 108 includes, for example, a network interface for enabling communications with a wired or wireless communications network. Additionally or alternatively, the communications circuitry 108 may include one or more network interface card(s), antenna(s), bus(es), switch(es), router(s), modem(s), and supporting hardware, firmware, and/or software, or any other device suitable for enabling communications via one or more communications network(s). In some embodiments, the communications circuitry 108 may include circuitry for interacting with an antenna(s) and/or other hardware or software to cause transmission of signals via the antenna(s) and/or to handle receipt of signals received via the antenna(s). In some embodiments, the communications circuitry 108 enables transmission to and/or receipt of data from a user device, one or more sensors, and/or other external computing device(s) in communication with the device 100.

In some embodiments, two or more of the sets of circuitry 102-108 are combinable. Alternatively, or additionally, one or more of the sets of circuitry 102-108 perform some or all of the operations and/or functionality described herein as being associated with another circuitry. In some embodiments, two or more of the sets of circuitry 102-108 are combined into a single module embodied in hardware, software, firmware, and/or a combination thereof.

In an example embodiment, the pressure adjustment circuitry 114 may be configured to execute instructions stored in the memory circuitry 104 or otherwise accessible to the processor for adjusting pressure sensor data based on tilt angle of the device 100 as described herein.

In an example embodiment, the fall detection circuitry 116 may be configured to execute instructions stored in the memory circuitry 104 or otherwise accessible to the processor for using the adjusted pressure sensor data to detect if a user of the device 100 has fallen.

Reference will now be made to FIG. 2, which provides a flowchart illustrating example steps, processes, procedures, and/or operations in accordance with various embodiments of the present disclosure. Various methods described herein, including, for example, example methods as shown in FIG. 2, may provide various technical benefits and improvements. It is noted that each block of the flowchart, and combinations of blocks in the flowchart, may be implemented by various means such as hardware, firmware, circuitry and/or other devices associated with execution of software including one or more computer program instructions. For example, one or more of the procedures described in FIG. 2 may be embodied by computer program instructions, which may be stored by a non-transitory memory of an apparatus employing an embodiment of the present disclosure and executed by a processor in the apparatus. These computer program instructions may direct a computer or other programmable apparatus to function in a particular manner, such that the instructions stored in the computer-readable storage memory produce an article of manufacture, the execution of which implements the function specified in the flowchart block(s).

As described above and as will be appreciated based on this disclosure, embodiments of the present disclosure may be configured as methods, mobile devices, backend network devices, and the like. Accordingly, embodiments may comprise various means including entirely of hardware or any combination of software and hardware. Furthermore, embodiments may take the form of a computer program product on at least one non-transitory computer-readable storage medium having computer-readable program instructions (e.g., computer software) embodied in the storage medium. Similarly, embodiments may take the form of a computer program code stored on at least one non-transitory computer-readable storage medium. Any suitable computer-readable storage medium may be utilized including non-transitory hard disks, CD-ROMs, flash memory, optical storage devices, or magnetic storage devices.

Referring now to FIG. 2, an example flow diagram illustrating an example method 200 for adjusting sensor data from waterproof sensors based on a tilt angle of the device in accordance with some embodiments of the present disclosure is illustrated. In some embodiments, the example method 200 may be implemented by an example device described herein, including, but not limited to, the example device 100 described above in connection with FIG. 1.

The example method 200 shown in FIG. 2 starts at step/operation 202. At step/operation 202, a processor (such as, but not limited to, the processing circuitry 102 of the device 100 described above in connection with FIG. 1) determines a tilt angle of the device. In various embodiments, the tilt angle of the device is determined using data received from an accelerometer (such as, but not limited to, the accelerometer 112 of the device 100 described above in connection with FIG. 1) using conventionally known calculations, such as by using the zero-degree axis (i.e., the axis that is the same direction as gravity, which is often termed the Z-axis) data from the accelerometer.

At step/operation 204, a processor (such as, but not limited to, the processing circuitry 102 of the device 100 described above in connection with FIG. 1) receives a pressure value from a pressure sensor (such as, but not limited to, the pressure sensor 110 of the device 100 described above in connection with FIG. 1). This pressure value may be termed an unadjusted pressure value, as this value has not yet been adjusted based on the tilt angle of the device.

At step/operation 206, a processor (such as, but not limited to, the processing circuitry 102 and/or the pressure adjustment circuitry 114 of the device 100 described above in connection with FIG. 1) determines a pressure adjustment value that will be used to determine an adjusted pressure value (described further below). In various embodiments, the pressure adjustment value is based on (a) the tilt angle determined at step/operation 202 and (b) a slope value of pressure test data taken at each of two different test angles. In some embodiments, the two test angles are a zero degree test angle and a 180 degree test angle, although different test angles could be used.

In various embodiments, the pressure test data is determined during the manufacturing process. In various embodiments, a plurality of pressure readings (e.g., about 100) are taken when the device is positioned at zero degrees, those readings are averaged, a plurality of pressure readings (e.g., about 100) are taken when the device is positioned at 180 degrees, and those readings are averaged. In various embodiments, the difference between the average of the pressure readings at zero degrees and the average of the pressure readings at 180 degrees is calculated.

In various embodiments, a slope value is calculated based on the average of the pressure test readings taken at the zero degree test angle and on the average of the pressure test readings taken at the 180 degree test angle. Specifically, in some embodiments, the slope value is calculated as the average of the pressure test readings taken at the 180 degree test angle minus the average of the pressure test readings taken at the zero degree test angle, divided by 180. If two different test angles are used (instead of zero and 180 degrees), the slope value is calculated as the average of the pressure test readings taken at the second test angle minus the average of the pressure test readings taken at the first test angle, divided by the difference between the second and first test angles. In an example embodiment in which the average pressure at 180 degrees is 0.15 hPA less than the pressure at zero degrees, the slope b = -0.15 / 180 = -0.000833.

In various embodiments, the pressure adjustment value is determined at step/operation 206 by multiplying the tilt angle determined at step/operation 202 by the slope value determined during testing of the pressure sensor.

At step/operation 208, a processor (such as, but not limited to, the processing circuitry 102 and/or the pressure adjustment circuitry 114 of the device 100 described above in connection with FIG. 1) determines an adjusted pressure value using the pressure adjustment value determined at step/operation 206. In various embodiments, the adjusted pressure value is determined by adding the pressure adjustment value determined at step/operation 206 to the unadjusted pressure value received at step/operation 204.

In various embodiments, the example method 200 runs continuously on the device, or the example method 200 may run only when, for example, a fall detection option is activated.

In various embodiments, the adjusted pressure value determined at step/operation 208 is used in a fall detection algorithm. In various embodiments, such a fall detection algorithm issues an alert if it is determined that the user of the device has fallen. In various embodiments, such a fall detection algorithm determines if a user of the device has fallen if a change in the adjusted pressure value exceeds a predetermined threshold.

### Conclusion

Many modifications and other embodiments of the disclosures set forth herein will come to mind to one skilled in the art to which these disclosures pertain having the benefit of teachings presented in the foregoing descriptions and the associated drawings. Although the figures only show certain components of the apparatus and systems described herein, it is understood that various other components may be used in conjunction with the system. Therefore, it is to be understood that the disclosures are not to be limited to the specific embodiments disclosed and that modifications and other embodiments are intended to be included within the scope of the appended claims. Moreover, the steps in the method described above may not necessarily occur in the order depicted in the accompanying diagrams, and in some cases one or more of the steps depicted may occur substantially simultaneously, or additional steps may be involved. Although specific terms are employed herein, they are used in a generic and descriptive sense only and not for purposes of limitation.

While various embodiments in accordance with the principles disclosed herein have been shown and described above, modifications thereof may be made by one skilled in the art without departing from the spirit and the teachings of the disclosure. The embodiments described herein are representative only and are not intended to be limiting. Many variations, combinations, and modifications are possible and are within the scope of the disclosure. The disclosed embodiments relate primarily to fragmented wideband tympanometry techniques for true wireless stereo, however, one skilled in the art may recognize that such principles may be applied to any audio device. Alternative embodiments that result from combining, integrating, and/or omitting features of the embodiment(s) are also within the scope of the disclosure. Accordingly, the scope of protection is not limited by the description set out above.

Additionally, the section headings used herein are provided for consistency with the suggestions under 37 C.F.R. 1.77 or to otherwise provide organizational cues. These headings shall not limit or characterize the disclosure(s) set out in any claims that may issue from this disclosure.

While this detailed description has set forth some embodiments of the present disclosure, the appended claims cover other embodiments of the present disclosure which differ from the described embodiments according to various modifications and improvements. For example, the appended claims can cover any form of device which uses a waterproof pressure sensor or any type of sensor whose output may vary based on the tilt angle of the device, such as but not limited to mobile devices (e.g., mobile phones) and wearable devices (e.g., smart watches), robots and robotic devices, and electronic cigarettes.

Within the appended claims, unless the specific term "means for" or "step for" is used within a given claim, it is not intended that the claim be interpreted under 35 U.S.C. 112, paragraph 6.

Example embodiments of the present disclosure are summarized here. Other embodiments can also be understood from the entirety of the specification and the examples.

Example 1 : A device comprising:
- a pressure sensor;
- an accelerometer; and
- a controller configured to determine a tilt angle of the device, receive an unadjusted pressure value from the pressure sensor, determine a pressure adjustment value based on (a) the tilt angle and (b) a slope value of pressure test data taken at a first test angle and at a second test angle different than the first test angle, and determine an adjusted pressure value by adding the determined pressure adjustment value to the received unadjusted pressure value.

Example 2 : The device of example 1, wherein the controller determines the tilt angle of the device using data received from the accelerometer.

Example 3 : The device of example 1 or 2, wherein the slope value is based on an average of a plurality of pressure test readings taken at the first test angle and on an average of a plurality of pressure test readings taken at the second test angle.

Example 4 : The device of example 3, wherein the slope value is calculated as (the average of the plurality of pressure test readings taken at the second test angle minus the average of the plurality of pressure test readings taken at the first test angle) divided by a difference in degrees between the second test angle and the first test angle.

Example 5 : The device of any of examples 1 to 4, wherein the controller is further configured to repeatedly determine the tilt angle of the device, receive the unadjusted pressure value from the pressure sensor, determine the pressure adjustment value based on (a) the tilt angle and (b) the slope value of pressure test data taken at the first test angle and at the second test angle, and determine the adjusted pressure value by adding the determined pressure adjustment value to the received unadjusted pressure value.

Example 6 : The device of example 5, wherein the controller is further configured to determine if a user of the device has fallen based on changes in repeated determinations of the adjusted pressure value.

Example 7 : The device of example 6, wherein the controller determines that the user of the device has fallen if the changes in repeated determinations of the adjusted pressure value exceed a predetermined threshold.

Example 8 : The device of any of examples 1 to 7, wherein the device comprises a wearable device.

Example 9 : The device of any of examples 1 to 8, wherein the first test angle and the second test angle are 180 degrees apart.

Example 10 : The device of any of examples 1 to 9, wherein the first test angle is zero degrees and the second test angle is 180 degrees.

Example 11 : A method of adjusting a pressure reading from a pressure sensor in a device, the method comprising:
- determining a tilt angle of the device containing the pressure sensor;
- receiving an unadjusted pressure value from the pressure sensor;
- determining a pressure adjustment value based on (a) the tilt angle and (b) a slope value of pressure test data taken at a first test angle and at a second test angle different than the first test angle; and
- determining an adjusted pressure value by adding the determined pressure adjustment value to the received unadjusted pressure value.

Example 12 : The method of example 11, wherein the tilt angle of the device is determined using data received from an accelerometer of the device.

Example 13 : The method of example 11 or 12, wherein the slope value is based on an average of a plurality of pressure test readings taken at the first test angle and on an average of a plurality of pressure test readings taken at the second test angle.

Example 14 : The method of example 13, wherein the slope value is calculated as (the average of the plurality of pressure test readings taken at the second test angle minus the average of the plurality of pressure test readings taken at the first test angle) divided by a difference in degrees between the second test angle and the first test angle.

Example 15 : The method of any of examples 11 to 14, further comprising repeatedly performing the steps of:
- determining the tilt angle of the device;
- receiving the unadjusted pressure value from the pressure sensor;
- determining the pressure adjustment value based on (a) the tilt angle and (b) the slope value of pressure test data taken at the first test angle and at the second test angle; and
- determining the adjusted pressure value by adding the determined pressure adjustment value to the received unadjusted pressure value.

Example 16 : The method of example 15, further comprising determining if a user of the device has fallen based on changes in repeated determinations of the adjusted pressure value.

Example 17 : The method of example 16, wherein it is determined that the user of the device has fallen if the changes in repeated determinations of the adjusted pressure value exceed a predetermined threshold.

Example 18 : The method of any of examples 11 to 17, wherein the device comprises a wearable device.

Example 19 : The method of any of examples 11 to 18, wherein the first test angle and the second test angle are 180 degrees apart.
Example 20 : The method of any of examples 11 to 19, wherein first test angle is zero degrees and the second test angle is 180 degrees.

## Claims

1. A device comprising:
- a pressure sensor;
- an accelerometer; and
- a controller configured to determine a tilt angle of the device, receive an unadjusted pressure value from the pressure sensor, determine a pressure adjustment value based on (a) the tilt angle and (b) a slope value of pressure test data taken at a first test angle and at a second test angle different than the first test angle, and determine an adjusted pressure value by adding the determined pressure adjustment value to the received unadjusted pressure value.

2. A method of adjusting a pressure reading from a pressure sensor in a device, the method comprising:
- determining a tilt angle of the device containing the pressure sensor;
- receiving an unadjusted pressure value from the pressure sensor;
- determining a pressure adjustment value based on (a) the tilt angle and (b) a slope value of pressure test data taken at a first test angle and at a second test angle different than the first test angle; and
- determining an adjusted pressure value by adding the determined pressure adjustment value to the received unadjusted pressure value.

3. The device of claim 1, wherein the controller determines, or the method of claim 2, wherein the tilt angle of the device using data received from the accelerometer or from an accelerometer of the device.

4. The device of claim 1 or 3, or the method of claim 2 or 3, wherein the slope value is based on an average of a plurality of pressure test readings taken at the first test angle and on an average of a plurality of pressure test readings taken at the second test angle.

5. The device or method of claim 4, wherein the slope value is calculated as (the average of the plurality of pressure test readings taken at the second test angle minus the average of the plurality of pressure test readings taken at the first test angle) divided by a difference in degrees between the second test angle and the first test angle.

6. The device of any of claims 1, 3 to 5, wherein the controller is further configured to perform, or the method of any of claims 2 to 5, further comprising repeatedly performing the steps of:
- determining the tilt angle of the device;
- receiving the unadjusted pressure value from the pressure sensor;
- determining the pressure adjustment value based on (a) the tilt angle and (b) the slope value of pressure test data taken at the first test angle and at the second test angle; and
- determining the adjusted pressure value by adding the determined pressure adjustment value to the received unadjusted pressure value.

7. The device of claim 6, wherein the controller is further configured to determine, or the method of claim 6, further comprising determining if a user of the device has fallen based on changes in repeated determinations of the adjusted pressure value.

8. The device of claim 7, wherein the controller determines, or the method of claim 7, wherein if is determined that the user of the device has fallen if the changes in repeated determinations of the adjusted pressure value exceed a predetermined threshold.

9. The device of any of claims 1, 3 to 8, or the method of any of claims 2 to 8, wherein the device comprises a wearable device.

10. The device of any of claims 1, 3 to 9, or the method of any of claims 2 to 9, wherein the first test angle and the second test angle are 180 degrees apart.

11. The device of any of claims 1, 3 to 10, or the method of any of claims 2 to 10, wherein the first test angle is zero degrees and the second test angle is 180 degrees.
